# EUROPÄISCHE PATENTSCHRIFT

(11) **EP 0 421 196 B1**
(45) Veröffentlichungstag und Bekanntmachung des Hinweises auf die Patenterteilung: **17.03.1993**
(21) Anmeldenummer: 90118079.4
(22) Anmeldetag: 20.09.1990
(51) Int. Cl.: C07C 29/141, C07C 29/145, C07C 31/125

(54) **Verfahren zur Herstellung von Alkoholen (einstufig)**
One-step process for the preparation of alcohols
Procédé de préparation d'alcools (en 1 étape)

(30) Priorität: 28.09.1989 DE 3932332
(43) Veröffentlichungstag der Anmeldung: 10.04.1991
(73) Patentinhaber: HOECHST AKTIENGESELLSCHAFT, 65926 Frankfurt am Main (DE)
(72) Erfinder: Horn, Gerhardt, Dr. Dipl.-Chem., D-4200 Oberhausen 11 (DE); Frohning, Carl Dieter, Dr. Dipl.-Chem., D-4230 Wesel (DE)

(56) Entgegenhaltungen:
- FR-A- 1 165 584
- SU-A- 759 121
- SU-A- 1 384 329
- US-A- 4 271 323

## Beschreibung

Die vorliegende Erfindung betrifft ein Verfahren zur Herstellung von Alkoholen ausgehend von organischen Carbonylverbindungen oder organische Carbonylverbindungen enthaltenden Gemischen, Es ist bekannt, organische Carbonylverbindungen bei erhöhter Temperatur und gegebenenfalls erhöhtem Druck mit Wasserstoff in Gegenwart eines Hydrierkatalysators zu den entsprechenden Alkoholen umzusetzen. Die Umsetzung kann sowohl diskontinuierlich als auch kontinuierlich in homogener oder heterogener Phase durchgeführt werden. Dementsprechend liegt der Hydrierkatalysator entweder in gelöster Form oder feinteilig als Suspension oder stückig als Festbettkontakt vor. Die zu hydrierenden Carbonylverbindungen können dem Katalysator in gasförmigem oder flüssigem Zustand zugeleitet werden.

Eine umfassende Darstellung bezüglich der Herstellung von Alkoholen durch katalytische Hydrierung von Carbonylverbindungen, insbesondere von Ketonen, Aldehyden und deren Derivaten findet sich in Houben-Wehl, Methoden der organischen Chemie, Georg Thieme Verlag Stuttgart-New York 1984, Band VI/1b Seiten 9 bis 111.

Die DE-AS 12 70 018 beschreibt ein Verfahren zur Herstellung von Alkoholen durch katalytische Hydrierung von Aldehyden in Gegenwart eines suspendierten Katalysators, wobei der gasförmige Wasserstoff der Reaktion in einer derartigen Geschwindigkeit zugesetzt wird, daß der Katalysator nur in einem Teil der flüssigen Phase suspendiert gehalten wird. Die vom Katalysator freie flüssige Phase wird kontinuierlich abgezogen. Zur Durchführung dieses Verfahrens wird Raney-Nickel als besonders geeigneter Katalysator verwendet.

Die DE-AS 12 31 227 betrifft ein Verfahren zur Herstellung von Alkoholen durch kontinuierliche Hydrierung von Aldehyden mit 6 bis 13 Kohlenstoffatomen in flüssiger Phase unter Verwendung eines amorphen Nickelkatalysators.

In der DE-OS 26 28 987 ist ein Verfahren zur Herstellung von Alkoholen mit 3 bis 5 Kohlenstoffatomen durch Umsetzung der entsprechenden Aldehyde in flüssiger Phase an einem Nickel, Kupfer und Mangan enthaltenden Trägerkatalysator beschrieben.

Die vorstehend beschriebenen Verfahren zur Herstellung von Alkoholen lassen hinsichtlich Umsatz und/oder Selektivität der katalytischen Hydrierreaktion Wünsche offen. Sie erfordern ferner relativ lange Reaktionszeiten und hohe Reaktionstemperaturen.

Es besteht daher Bedarf nach einem gegenüber dem Stand der Technik verbesserten Prozess. Die Aufgabe wird gelöst durch ein Verfahren zur Herstellung von Alkoholen durch Umsetzung organischer Carbonylverbindungen oder organische Carbonylverbindungen enthaltender Gemische mit Wasserstoff bei erhöhter Temperatur und gegebenenfalls erhöhtem Druck in Gegenwart eines Hydrierkatalysators.

Es ist dadurch gekennzeichnet, daß die Carbonylverbindungen oder die Gemische an einem Nickel, Aluminiumoxid und Zirkondioxid enthaltenden Trägerkatalysator bei 60 bis 150°C umgesetzt werden.

Gegenüber Verfahren gemäß dem Stand der Technik gestattet das erfindungsgemäße Verfahren, die Hydrierung von Carbonylverbindungen in zahlreichen Fällen nicht nur mit erhöhtem Umsatz, sondern auch mit gesteigerter Selektivität durchzuführen. Ferner erlaubt es im Vergleich mit bekannten Arbeitsweisen, die Hydrierung bei tieferen Temperaturen und/oder kürzeren Reaktionszeiten, d.h. höheren Durchsätzen ablaufen zu lassen.

Das erfindungsgemäße Verfahren läßt sich auf die Umsetzung von Ketonen, Ketonderivaten, Aldehyden und deren Derivate, anwenden.

Als Ketone können Aceton, Methylethylketon, Diethylketon, Hexanone, beispielsweise Cyclohexanon, Heptanone, Octanone, aber auch höhere Ketone sowie aromatische Ketone, wie Acetophenon, Benzophenon, als Ketonderivate Acetol (Hydroxyaceton), Acetoin (Acetylmethylcarbinol), Dihydroxyaceton, Benzoin, Lactone sowie Ketosen, wie Fructose eingesetzt werden.

Mit Hilfe des erfindungsgemäßen Verfahrens lassen sich aromatische, araliphatische, cycloaliphatische und aliphatische Aldehyde und deren Derivate, insbesondere cycloaliphatische und aliphatische, bevorzugt aliphatische Aldehyde und deren Derivate umsetzen. Besondere Bedeutung kommt dem Gebrauch aliphatischer geradkettiger und verzweigter Aldehyde mit 2 bis 18 C-Atomen zu. Mit Ausnahme von Acetaldehyd lassen sich diese Aldehyde beispielsweise durch Hydroformylierung von Olefinen herstellen. Sie können in vorgereinigter Form, aber auch als Rohprodukt, beispielsweise als rohes Hydroformylierungsprodukt in das erfindungsgemäße Verfahren eingesetzt werden.

Geeignete Aldehyde sind: Acetaldehyd, Propanal, n- und i-Butanal, n- und i-Pentanal, n- und i-Hexanal, n- und i-Heptanal, n- und i-Octanal, n- und i-Nonanal, n- und i-Decanal sowie n- und i-Alkanale mit 11 bis 18 Kohlenstoffatomen, insbesondere Acetaldehyd, Propanal, n- und i-Butanal, n- und i-Pentanal, n- und i-Octanal, n- und i-Nonanal sowie Alkanale mit 11 bis 18 Kohlenstoffatomen, bevorzugt Propanal, n- und i-Butanal, n- und i-Octanal sowie n- und i-Nonanal.

Erfindungsgemäß lassen sich auch ungesättigte Aldehyde umsetzen. Hierzu zählen: Acrolein, Crotonaldehyd, n- und i-Pentenal, n- und i-Hexenal, n- und i-Heptenal, n-und i-Octenal, insbesondere Acrolein, Crotonaldehyd, 2-Ethylhexenal, bevorzugt 2-Ethylhexenal.

Aldehydderivate sind Verbindungen, die sich von Aldehyden ableiten. Sie lassen sich durch eine Reihe üblicher Synthesen, wie Aldolisierung, Aldolkondensation, Substitutions- oder Additionsreaktionen - erwähnt sei die Anlagerung von Wasser an ungesättigte Aldehyde - herstellen und mit gutem Erfolg erfindungsgemäß in die entsprechenden Alkohole überführen.

Organische Carbonylverbindungen enthaltende Gemische sind Lösungen der vorstehend aufgezählten Carbonylverbindungen, insbesondere verdünnte Lösungen derselben, aber auch rohe Reaktionsprodukte, wie sie bei der Herstellung der Carbonylverbindungen, beispielsweise durch Aldolisierung, Aldolkondensation, Hydroformylierung, Substitution oder Addition, insbesondere in verdünntem Zustand anfallen.

Obgleich in derartigen Gemischen die Carbonylverbindungen häufig in herabgesetzter, mitunter sogar nur in recht geringer Konzentration enthalten sind, lassen sie sich erfindungsgemäß mit gutem Erfolg in die entsprechenden Alkohole überführen.

Der Hydrierkatalysator enthält 20 bis 90 Gew.-% Nickel bezogen auf Katalysatorzusammensetzung, sowie 1 bis 30, insbesondere 3 bis 15, bevorzugt 4 bis 10 Gew.-Teile Aluminiumoxid und 0,5 bis 20, insbesondere 1 bis 10, bevorzugt 1,5 bis 5 Gew.-Teile Zirkondioxid jeweils bezogen auf 100 Gew.-Teile Nickel als Copräzipitat auf einem Trägermaterial.

Da die Ausübung des erfindungsgemäßen Verfahrens an diesen besonderen Katalysator gebunden ist, soll im folgenden dessen Herstellung näher erläutert werden.

Man vermischt eine wäßrige Ni-Al-Zr-Mischsalzlösung mit einer wäßrigen Lösung einer basischen Verbindung als Fällungsmittel, setzt die basische Verbindung in einem stöchiometrischen Überschuß von 5 bis 100 %, bezogen auf die zur quantitativen Fällung von Ni, Al und Zr erforderliche Menge, ein, fällt bei 60 bis 120°C und pH 7 bis 10 gleichzeitig Ni, Al und Zr und scheidet sie als Copräzipitat auf einem Trägermaterial ab.

Um einer unerwünschten Hydrolyse vorzubeugen und die Fällung günstig zu beeinflussen, empfiehlt es sich, der Mischsalzlösung freie Säure im Überschuß entsprechend einem Verhältnis H⁺ : Zr⁴⁺ = (2 bis 40) : 1, insbesondere (3 bis 30) : 1, bevorzugt (4 bis 20) : 1, zuzufügen. Die freie Säure wird durch Titration mit NaOH (Endpunkt pH = 0,8) bestimmt.

Als freie Säure können Salzsäure, Schwefelsäure und bevorzugt Salpetersäure eingesetzt werden.

Die Mischsalzlösung besteht aus 10 bis 100, insbesondere 20 bis 80, bevorzugt 30 bis 50 g Ni/l. Sie weist Aluminium entsprechend 1 bis 30, insbesondere 3 bis 15, bevorzugt 4 bis 10 Gew.-Teilen Al₂O₃ je 100 Gew.-Teile Ni auf. Ferner enthält sie Zirkon entsprechend 0,5 bis 20, insbesondere 1 bis 10, bevorzugt 1,5 bis 5 Gew.-Teilen ZrO₂ je 100 Gew.-Teile Ni.

Die Mischsalzlösung stellt man her, indem man wasserlösliche anorganische, organische oder komplexe Salze von Nickel, Zirkon und Aluminium, insbesondere deren Sulfate, Chloride, Acetate und Nitrate, bevorzugt deren Nitrate in Wasser löst.

Als Fällungsmittel dient eine wäßrige Lösung einer basischen Verbindung, insbesondere eine wäßrige Alkalicarbonat-, Alkalihydrogencarbonat-, Alkalihydroxid-, Ammoniumhydroxid- oder Ammoniumcarbonatlösung, die einen pH-Wert von 7,5 bis 13, insbesondere 8 bis 12, bevorzugt 9 bis 11, aufweist.

Recht gute Ergebnisse liefern wäßrige Losungen mit 0,3 bis 1,5, insbesondere 0,8 bis 1,2 Mol Alkalicarbonat/l Lösung.

Um eine möglichst vollständige Fällung eines besonders homogenen Copräzipitats sicherzustellen, setzt man die basische Verbindung im stöchiometrischen Überschuß von 5 bis 100, insbesondere 10 bis 70, bevorzugt 20 bis 40%, jeweils bezogen auf die zur vollständigen Fällung von Ni, Al und Zr erforderliche Menge basische Verbindung, ein.

Die Fällung wird herbeigeführt, indem man entweder die Mischsalzlösung und das Fällungsmittel kontinuierlich zusammenführt und vermischt oder, gemäß einer bevorzugten Variante, das Fällungsmittel vorlegt und die Mischsalzlösung in das Fällungsmittel einleitet.

Das Trägermaterial kann mit der Mischsalzlösung und/oder mit dem Fällungsmittel in die Reaktion eingesetzt werden.

Es hat sich als besonders vorteilhaft erwiesen, zunächst die Mischsalzlösung und das Fällungsmittel miteinander zu vermischen und anschließend das Trägermaterial zuzusetzen.

Als Trägermaterial eignen sich Aktivkohle, Tonerden, Bimsstein, γ-Al₂O₃, SiO₂, Kieselgel, Kieselgur und Kieselerden. Besonders haben sich SiO₂, Kieselgel, Kieselgur und Kieselerde bewährt. Bevorzugt werden Kieselgur und SiO₂ in Form gefällter Kieselsäure eingesetzt.

Üblicherweise setzt man 6 bis 80, insbesondere 15 bis 65, bevorzugt 35 bis 50 Gew.-Teile Trägermaterial je 100 Gew.-Teile Ni ein.

Zur Herstellung homogener Copräzipitate halt man während der Fällung einen pH-Bereich von 7 bis 10, insbesondere 7,3 bis 9, bevorzugt 7,5 bis 8,5 und eine Temperatur von 60 bis 120, insbesondere 70 bis 110, bevorzugt 95 bis 105°C ein.

Nach Beendigung der Fällung wird gegebenenfalls nach Abkühlung filtriert, gewaschen, erforderlichenfalls geformt, anschließend getrocknet und reduziert.

Die Trocknung wird in einem Temperaturbereich zwischen 40 bis 120, insbesondere 50 bis 100 °C durchgeführt.

Die Reduktion mittels Wasserstoff erfolgt bei 300 bis 550 °C, wobei ein Reduktionsgrad von wenigstens 80 %, insbesondere wenigstens 90 %, bevorzugt 95 % und darüber anzustreben ist. Unter Reduktionsgrad versteht man Anteil Nickelmetall: Gesamtgehalt Nickel x 100 %.

Das erfindungsgemäße Verfahren zur Herstellung von Alkoholen durch Umsetzung von organischen Carbonylverbindungen mit Wasserstoff in Gegenwart des zuvor beschriebenen Katalysators kann diskontinuierlich oder kontinuierlich durchgeführt werden.

Arbeitet man in flüssiger Phase, so kann der Katalysator entweder als feinteilige Suspension oder als stückiger Festbettkontakt eingesetzt werden. Die Reaktionstemperatur beträgt 60 bis 150°C. Will man besonders schonend arbeiten, so wendet man eine Reaktionstemperatur von 60 bis 80°C an. In den meisten Fällen läßt sich die erfindungsgemäße Hydrierung bei Temperaturen von 80 bis 140°C, insbesondere 90 bis 130°C, bevorzugt 100 bis 125°C durchführen. Der Druck liegt üblicherweise bei 0,1 bis 25, insbesondere 1,0 bis 15, bevorzugt 2,0 bis 10 MPa.

Man leitet die umzusetzende Carbonylverbindung entweder dem suspendierten Katalysator in flüssiger Form zusammen mit Wasserstoff absatzweise oder kontinuierlich zu oder führt den die Carbonylverbindung aufweisenden Einsatzstoff in Gleich- oder Gegenstrom mit Wasserstoff über den in stückiger Form vorliegenden, als Festbett angeordneten Nickel, Aluminiumoxid und Zirkondioxid enthaltenden Trägerkatalysator. Bei technischer Realisierung des erfindungsgemäßen Verfahrens dürfte die Festbettfahrweise häufig bevorzugt werden, wobei das Einsatzgemisch entweder von oben nach unten (Rieselfahrweise) oder von unten nach oben (Sumpffahrweise) über den festangeordneten Katalysator geleitet wird. Wendet man die Rieselfahrweise an, so leitet man den Wasserstoff im Gleich- oder Gegenstrom, bevorzugt im Gleichstrom zum Einsatzmaterial, will man hingegen die Sumpffahrweise praktizieren, so führt man vorteilhaft den Wasserstoff im Gleichstrom zum Einsatzgemisch über den stückigen, festangeordneten Trägerkatalysator.

Bei kontinuierlicher Durchführung des Verfahrens in flüssiger Phase beträgt die Raumgeschwindigkeit, ausgedrückt als Volumen flüssiger Einsatzstoff/Volumen Katalysator x Stunde (V/Vh), 0,6 bis 2,0, insbesondere 0,8 bis 1,6, bevorzugt 1,0 bis 1,5.

Wasserstoff muß mindestens entsprechend der Stöchiometrie der Umsetzung eingesetzt werden. In der Regel wird man jedoch einen stöchiometrischen Überschuß an Wasserstoff verwenden, um die Reaktion günstig zu beeinflussen. Ein Wasserstoffüberschuß von 1 bis 100, insbesondere 2 bis 50, bevorzugt 5 bis 10 Mol je Äquivalent Carbonylverbindungen genügt zur Durchführung der Hydrierung in flüssiger Phase. Nicht umgesetzter Wasserstoff kann in die Reaktion zurückgeführt werden.

Das erfindungsgemäße Verfahren eignet sich aber auch zur Durchführung in der Gasphase, wobei das Einsatzmaterial in gasförmigem Zustand zusammen mit Wasserstoff über den stückigen, festangeordneten Nickel, Aluminiumoxid und Zirkondioxid enthaltenden Trägerkatalysator geführt wird.

Die Umsetzung in der Gasphase wird bei 60 bis 150°C durchgeführt. In den meisten Fällen genügt eine Temperatur von 80 bis 140, insbesondere 90 bis 130°C. Der Druck beträgt 0,05 bis 2,0, insbesondere 0,1 bis 1,2, bevorzugt 0,15 bis 1,0 MPa.

Bei kontinuierlicher Durchführung des Verfahrens in der Gasphase beträgt die Raumgeschwindigkeit, ausgedrückt als Volumen flüssiger Einsatzstoff/Volumen Katalysator x Stunde (V/Vh), 0,2 bis 1,5, insbesondere 0,3 bis 1,2, bevorzugt 0,5 bis 1,0.

Wasserstoff muß mindestens entsprechend der Stöchiometrie der Umsetzung eingesetzt werden. Üblicherweise verwendet man jedoch einen stöchiometrischen Überschuß an Wasserstoff, um die Reaktion in die gewünschte Richtung zu lenken. Ein Wasserstoffüberschuß von 0,5 bis 50, insbesondere 1 bis 20, bevorzugt 2 bis 10 Mol je Äquivalent Carbonylverbindung erweist sich für die Gasphasehydrierung als ausreichend. Nichtumgesetzter Wasserstoff kann der Reaktion wieder zugeführt werden.

Welcher Arbeitsweise man sich bedient, hängt einerseits von der Art der Carbonylverbindung, andererseits aber auch von den zur Verfügung stehenden Apparaturen ab, in denen die Umsetzung durchgeführt werden soll. Eine generelle Empfehlung, einer der vorstehend erläuterten Verfahrensvarianten den Vorzug zu geben, kann in Hinblick auf die breite Anwendungsmöglichkeit des erfindungsgemäßen Verfahrens nicht ausgesprochen werden.

Die nachfolgend aufgeführten Beispiele belegen die vorliegende Erfindung, ohne sie zu begrenzen.

### Experimenteller Teil

### Beispiel 1

In einen mit einem Magnethubrührer versehenen Autoklaven (Volumen 1000 ml) werden 400 g n-Butanal und 2,4 g eines Katalysators, der 100 Gew.-Teile Ni, 5 Gew.-Teile Aluminiumoxid sowie 3 Gew.-Teile Zirkondioxid als Copräzipitat und 40 Gew.-Teile SiO₂ als Träger enthält, unter Ausschluß von Luft vorgelegt. Anschließend wird Wasserstoff aufgedrückt, unter Rühren aufgeheizt und durch Zugabe von Wasserstoff der gewünschte Druck eingestellt. Die Reaktion wird abgebrochen, sobald keine Wasserstoffaufnahme mehr erfolgt (Hydrierzeit).

### Reaktionsbedingungen

- Druck: 7,0 MPa
- Temperatur: 115°C
- Hydrierzeit: 70 Min

Das Hydrierprodukt weist eine CO-Zahl von lediglich 1,2 (mg KOH/g) entsprechend 0,15 Gew.-% n-Butanal auf.

### Vergleichsbeispiel 1

Es wird wie in Beispiel 1 gearbeitet, jedoch ein üblicher Katalysator mit etwa 55 Gew.-% Ni und etwa 30 bis 35 Gew.-% SiO₂,
der allerdings weder Aluminiumoxid noch Zirkondioxid enthält, verwendet.

### Reaktionsbedingungen

- Druck: 7,0 MPa
- Temperatur: 115°C
- Hydrierzeit: 100 bis 120 Min

Das Hydrierprodukt weist eine CO-Zahl von 1,5 (mg KOH/g) entsprechend 0,2 Gew.-% n-Butanal auf.

### Beispiel 2

In einen mit einem Magnethubrührer versehenen Autoklaven (Volumen 1000 ml) werden 400 g eines Gemisches, das entsprechend einer CO-Zahl von 126 (mg KOH/g) etwa 29 Gew.-% Octanal (hergestellt durch Hydroformylierung von Hepten) sowie ungesattigte Verbindungen entsprechend einer Iodzahl von 16,1 (g J₂/100g) enthält, und 12,5 g des in Beispiel 1 verwendeten Katalysators vorgelegt. Anschließend wird, wie in Beispiel 1 angegeben, verfahren.

### Reaktionsbedingungen

- Druck: 8,0 MPa
- Temperatur: 120°C
- Hydrierzeit: 60 Min

Das Hydrierprodukt weist eine CO-Zahl von lediglich 0,5 (mg KOH/g) entsprechend 0,1 Gew.-% Octanal und eine Iodzahl von 2,5 (g J₂/100g) auf.

### Vergleichsbeispiel 2

Es wird wie in Beispiel 2 gearbeitet, jedoch ein üblicher Katalysator mit etwa 65 Gew.-% Ni,
der allerdings kein Nickel-Aluminiumoxid-Zirkondioxid-Copräzipitat enthält, verwendet.

### Reaktionsbedingungen

- Druck: 8,0 MPa
- Temperatur: 120°C
- Hydrierzeit: 75 Min

Das Hydrierprodukt weist eine CO-Zahl von 3,2 (mg KOH/g) entsprechend 0,8 Gew.-% Octanal und eine Iodzahl von 12,5 (g J₂/100g) auf.

### Beispiel 3

In einen mit einem Magnethubrührer versehenen Autoklaven (Volumen 1000 ml) werden 400 g eines Gemisches, das entsprechend einer CO-Zahl von 234 (mg KOH/g) etwa 59 Gew.-% i-Nonanal (hergestellt durch Hydroformylierung von Diisobutylen) sowie ungesättigte Verbindungen entsprechend einer Iodzahl von 36 (g J₂/100g) enthält, und 7,5 g des in Beispiel 1 verwendeten Katalysators vorgelegt. Anschließend wird, wie in Beispiel 1 angegeben, verfahren.

### Reaktionsbedingungen

- Druck: 8,0 MPa
- Temperatur: 120°C
- Hydrierzeit: 60 bis 70 Min

Das Hydrierprodukt weist eine CO-Zahl von lediglich 0,2 bis 0,3 (mg KOH/g) entsprechend etwa 0,1 Gew.-% i-Nonanal und eine Iodzahl von lediglich 0,2 bis 0,3 (g J₂/100g) auf.

### Vergleichsbeispiel 3

Es wird wie in Beispiel 3 gearbeitet, jedoch der in Vergleichsbeispiel 1 verwendete Katalysator eingesetzt.

### Reaktionsbedingungen

- Druck: 8,0 MPa
- Temperatur: 120°C
- Hydrierzeit: 85 Min

Das Hydrierprodukt weist eine CO-Zahl von 0,5 bis 0,6 (mg KOH/g) entsprechend 0,2 bis 0,3 Gew.-% i-Nonanal und eine Iodzahl von 12 (g J₂/100g) auf.

### Beispiel 4

In einen mit einem Magnethubrührer versehenen Autoklaven (Volumen 1000 ml) werden 400 g eines Gemisches, das neben 1,2 Gew.-% n-Propanol, 8,1 Gew.-% Nebenprodukten und etwa 83 Gew.-% (i-Butanol + Wasser) noch 7,5 Gew.-% β-Hydroxypropanal (ausgewiesen durch gaschromatografische Analyse) enthält, und 6,7 g des in Beispiel 1 verwendeten Katalysators vorgelegt. Anschließend wird, wie in Beispiel 1 angegeben, verfahren.

### Reaktionsbedingungen

- Druck: 8,0 MPa
- Temperatur: 90°C
- Hydrierzeit: 40 Min

Das Hydrierprodukt weist eine CO-Zahl von 0,78 (mg KOH/g) entsprechend <0,1 Gew.-% β-Hydroxypropanal auf. Es setzt sich laut gaschromatografischer Analyse - wie folgt - zusammen:
- Vorlauf: 0,1 Gew.-%
- n-Propanol: 1,3 Gew.-%
- Nebenprodukte: 7,3 Gew.-%
- β-Hydroxypropanal: <0,1 Gew.-%
- Propandiol-1,3: 7,7 Gew.-%
- i-Butanol + Wasser: 83,5 Gew.-%

### Vergleichsbeispiel 4

Es wird wie in Beispiel 4 gearbeitet, jedoch der in Vergleichsbeispiel 1 verwendete Katalysator eingesetzt.

### Reaktionsbedingungen

- Druck: 8,0 MPa
- Temperatur: 90°C
- Hydrierzeit: 210 Min

Das Hydrierprodukt setzt sich laut gaschromatografischer Analyse - wie folgt - zusammen:
- Vorlauf: 0,1 Gew.-%
- n-Propanol: 1,4 Gew.-%
- Nebenprodukte: 9,4 Gew.-%
- β-Hydroxypropanal: <0,1 Gew.-%
- Propandiol-1,3: 7,3 Gew.-%
- i-Butanol + Wasser: 81,8 Gew.-%

Wie ein Vergleich von Beispiel 4 und Vergleichsbeispiel 4 zeigt, erfolgt die Umsetzung des β-Hydroxypropanals erfindungsgemäß in wesentlich kürzerer Zeit bei gleichzeitig geringerer Bildung von Nebenprodukten.

### Beispiel 5

In einen mit einem Magnethubrührer versehenen Autoklaven (Volumen 1000 ml) werden 400 g eines Gemisches, das überwiegend n-Butanol und entsprechend einer CO-Zahl von 12,7 (mg KOH/g) 1,6 Gew.-% n-Butanal sowie ungesättigte Verbindungen entsprechend einer Iodzahl von 2 bis 4 (g J₂/100g) enthält, und 25 g des in Beispiel 1 verwendeten Katalysators vorgelegt. Anschließend wird, wie in Beispiel 1 angegeben, verfahren.

### Reaktionsbedingungen

- Druck: 8,0 MPa
- Temperatur: 115°C
- Hydrierzeit: 120 Min

Das Hydrierprodukt weist eine CO-Zahl von 0,04 (mg KOH/g) entsprechend 0,005 Gew.-% n-Butanal und eine Iodzahl von 0,01 (g J₂/100g) auf.

### Vergleichsbeispiel 5

Es wird wie in Beispiel 5 gearbeitet, jedoch der in Vergleichsbeispiel 1 verwendete Katalysator eingesetzt.

### Reaktionsbedingungen

- Druck: 8,0 MPa
- Temperatur: 115°C
- Hydrierzeit: 120 Min

Das Hydrierprodukt weist eine CO-Zahl von 1,95 (mg KOH/g) entsprechend 0,25 Gew.-% n-Butanal und eine Iodzahl von 0,17 (g J₂/100g) auf.

### Beispiel 6 (kontinuierliche Hydrierung in flüssiger Phase)

In einem senkrecht stehenden Rohr befindet sich eine Schüttung von 1000 ml des in Beispiel 1 verwendeten Katalysators (tablettierte Form). Je Stunde werden 1000 ml eines flüssigen Gemisches, das überwiegend n-Butanol und entsprechend einer CO-Zahl von 10 bis 13 (mg KOH/g) 1,3 bis 1,8 Gew.-% n-Butanal sowie ungesättigte Verbindungen entsprechend einer Iodzahl von 1,5 bis 2,4 (g J₂/100g) enthält, im Gleichstrom mit Wasserstoff von unten nach oben über die Katalysatorschüttung geleitet.

### Reaktionsbedingungen

- Druck: 8,0 MPa
- Temperatur: 105°C
- Wasserstoff: 100 Nl/h

Das Hydrierprodukt weist eine CO-Zahl von 0,1 bis 0,5, entsprechend 0,012 bis 0,06 Gew.-% n-Butanal und eine Iodzahl von 0,01 (g J₂/100g) auf.

### Vergleichsbeispiel 6 (kontinuierliche Hydrierung in flüssiger Phase)

Es wird wie in Beispiel 6 gearbeitet, wobei sich in dem senkrecht stehenden Rohr eine Schüttung von 1000 ml des in Vergleichsbeispiel 1 verwendeten Katalysators (tablettierte Form) befindet. Es wird dasselbe Einsatzgemisch wie in Beispiel 6 über die Schüttung, allerdings lediglich in einer Menge von 250 bis 300 ml je Stunde, geleitet.

### Reaktionsbedingungen

- Druck: 8,0 MPa
- Temperatur: 120°C
- Wasserstoff: 25 bis 30 Nl/h

Das Hydrierprodukt weist eine CO-Zahl von 0,6 bis 1,6 (mg KOH/g), entsprechend 0,08 bis 0,2 Gew.-% n-Butanal auf.

Wie Beispiel 6 im direkten Vergleich zeigt, gestattet das erfindungsgemäße Verfahren einen 3 bis 4 fach höheren Durchsatz bei gleichzeitig besserem Umsatz (belegt durch die CO-Zahl) und niedrigerer Reaktionstemperatur.

### Beispiel 7 (kontinuierliche Hydrierung in flüssiger Phase)

In einem senkrecht stehenden Rohr befindet sich eine Schüttung von 1000 ml des in Beispiel 1 verwendeten Katalysators (tablettierte Form). Je Stunde werden 1100 ml eines flüssigen Gemisches, das überwiegend 2-Ethylhexanol und entsprechend einer CO-Zahl von 4,9 (mg KOH/g) 1,1 Gew.-% (2-Ethylhexanal + 2-Ethylhexenal) sowie ungesättigte Verbindungen (2-Ethylhexenal + andere ungesättigte Verbindungen) entsprechend einer Iodzahl von 1,6 (g J₂/100g) enthält, im Gleichstrom mit Wasserstoff von oben nach unten über die Katalysatorschüttung geleitet.

### Reaktionsbedingungen

- Druck: 2,5 MPa
- Temperatur: 115°C
- Wasserstoff: 120 Nl/h

Das Hydrierprodukt weist eine CO-Zahl von 0,041 (mg KOH/g) entsprechend 0,009 Gew.-% (2-Ethylhexanal + 2-Ethylhexenal) und eine Iodzahl von 0,028 (g J₂/100g) auf.

### Vergleichsbeispiel 7 (kontinuierliche Hydrierung in flüssiger Phase)

Es wird wie in Beispiel 7 gearbeitet, wobei sich in dem senkrecht stehenden Rohr eine Schüttung von 1000 ml des im Vergleichsbeispiel 1 verwendeten Katalysators (tablettierte Form) befindet. Je Stunde werden 700 ml eines flussigen Gemisches, das überwiegend 2-Ethylhexanol und entsprechend einer CO-Zahl von 3,0 bis 3,7 (mg KOH/g) 0,68 bis 0,84 Gew.-% (2-Ethylhexanal + 2-Ethylhexenal) sowie ungesättigte Verbindungen (2-Ethylhexenal + andere ungesättigte Verbindungen) entsprechend einer Iodzahl von 1,6 (g J₂/100g) enthält, im Gleichstrom mit Wasserstoff von oben nach unten über die Katalysatorschüttung geleitet.

### Reaktionsbedingungen

- Druck: 2,5 MPa
- Temperatur: 130°C
- Wasserstoff: 76 Nl/h

Wie Beispiel 7 in direktem Vergleich zeigt, gestattet das erfindungsgemäße Verfahren einen deutlich höheren Durchsatz (nämlich V/Vh 1,1 statt V/Vh 0,7) und führt trotz niedrigerer Reaktionstemperatur zu einer verbesserten Produktqualität (belegt durch CO-Zahl und Iodzahl).

### Beispiel 8 (kontinuierliche Hydrierung in der Gasphase)

In einem senkrecht stehenden Rohr befindet sich eine Schüttung von 1000 ml des in Beispiel 1 verwendeten Katalysators (tablettierte Form). Je Stunde werden 500 ml eines Gemisches, das 65 bis 75 Gew.-% n- und i-Butanal, 8 bis 15 Gew.-% n- und i-Butanol, 3,5 bis 5,5 Gew.-% n-und i-Butylformiat und 1 bis 3 Gew.-% n- und i-Dibutylether (ausgewiesen durch gaschromatografische Analyse) enthält, einem Verdampfer zugeführt. Das Gemisch wird in gasförmigem Zustand im Gleichstrom mit Wasserstoff von oben nach unten über die Katalysatorschüttung geleitet.

### Reaktionsbedingungen

- Druck:: 0,2 MPa
- Temperatur:: 90°C
- V/Vh*:: 0,5
- Wasserstoff:: 2200 Nl/h

*Raumgeschwindigkeit (Volumen flüssiger Einsatzstoff/Volumen Katalysator x Stunde)

Das Hydrierprodukt weist laut gaschromatografischer Analyse 0,2 bis 0,4 Gew.-% n- und i-Butanal, 96,3 bis 98,1 Gew.-% n- und i-Butanol, 1,2 bis 1,8 Gew.-% n- und i-Butylformiat und 0,5 bis 1,5 Gew.-% n- und i-Dibutylether auf.

## Patentansprüche

1. Verfahren zur Herstellung von Alkoholen durch Umsetzung organischer Carbonylverbindungen oder organische Carbonylverbindungen enthaltender Gemische mit Wasserstoff bei erhöhter Temperatur und gegebenenfalls erhöhtem Druck in Gegenwart eines Hydrierkatalysators, dadurch gekennzeichnet, daß die Carbonylverbindungen oder die Gemische an einem Nickel, Aluminiumoxid und Zirkondioxid enthaltenden Trägerkatalysator bei 60 bis 150°C umgesetzt werden.

2. Verfahren nach Anspruch 1, dadurch gekennzeichnet, daß als Carbonylverbindungen Ketone, Ketonderivate, Aldehyde und Aldehydderivate eingesetzt werden.

3. Verfahren nach einem oder mehreren der Ansprüche 1 bis 2, dadurch gekennzeichnet, daß als Carbonylverbindungen Aldehyde und Aldehydderivate, insbesondere aliphatische Aldehyde und deren Derivate eingesetzt werden.

4. Verfahren nach einem oder mehreren der Ansprüche 1 bis 3, dadurch gekennzeichnet, daß der Hydrierkatalysator 20 bis 90 Gew.-% Nickel bezogen auf Katalysatorzusammensetzung, sowie 1 bis 30 Gew.-Teile Aluminiumoxid und 0,5 bis 20 Gew.-Teile Zirkondioxid jeweils bezogen auf 100 Gew.-Teile Ni als Copräzipitat auf einem Trägermaterial enthält.

5. Verfahren nach einem oder mehreren der Ansprüche 1 bis 4, dadurch gekennzeichnet, daß das Trägermaterial Aktivkohle, Tonerde, Bimsstein, γ-Al₂O₃, SiO₂, Kieselgel, Kieselgur und/oder Kieselerde, insbesondere SiO₂, Kieselgel, Kieselgur und/oder Kieselerde, bevorzugt Kieselgur und/oder SiO₂ in Form gefällter Kieselsäure ist.

6. Verfahren nach einem oder mehreren der Ansprüche 1 bis 5, dadurch gekennzeichnet, daß der Hydrierkatalysator 6 bis 80, insbesondere 15 bis 65, bevorzugt 35 bis 50 Gew.-Teile Trägermaterial je 100 Gew.-Teile Ni enthält.

7. Verfahren nach einem oder mehreren der Ansprüche 1 bis 6, dadurch gekennzeichnet, daß die Umsetzung in flüssiger Phase bei 60 bis 150, insbesondere 80 bis 140, bevorzugt 90 bis 130, besonders bevorzugt 100 bis 125°C und unter einem Druck von 0,1 bis 25, insbesondere 1,0 bis 15, bevorzugt 2,0 bis 10 MPa durchgeführt wird.

8. Verfahren nach Anspruch 7, dadurch gekennzeichnet, daß die Raumgeschwindigkeit, ausgedrückt als Volumen flüssiger Einsatzstoff/Volumen Katalysator x Stunde (V/Vh), 0,6 bis 2,0, insbesondere 0,8 bis 1,6, bevorzugt 1,0 bis 1,5 beträgt.

9. Verfahren nach einem oder mehreren der Ansprüche 1 bis 6, dadurch gekennzeichnet, daß die Umsetzung in gasförmiger Phase bei 60 bis 150, insbesondere 80 bis 140, bevorzugt 90 bis 130°C und unter einem Druck von 0,05 bis 2,0, insbesondere 0,1 bis 1,2, bevorzugt 0,15 bis 1,0 MPa durchgeführt wird.

10. Verfahren nach Anspruch 9, dadurch gekennzeichnet, daß die Raumgeschwindigkeit, ausgedrückt als Volumen flüssiger Einsatzstoff/Volumen Katalysator x Stunde (V/Vh), 0,2 bis 1,5, insbesondere 0,3 bis 1,2, bevorzugt 0,5 bis 1,0 beträgt.

## Claims

1. A process for the preparation of alcohols by reaction of organic carbonyl compounds or mixtures containing organic carbonyl compounds with hydrogen in the presence of a hydrogenation catalyst at elevated temperature and if appropriate under increased pressure, which comprises reacting the carbonyl compounds or the mixtures at 60 to 150°C over a supported catalyst containing nickel, aluminum oxide and zirconium dioxide.

2. The process as claimed in claim 1, wherein ketones, ketone derivatives, aldehydes and aldehyde derivatives are employed as the carbonyl compounds.

3. The process as claimed in either of claims 1 and 2, wherein aldehydes and aldehyde derivatives, in particular aliphatic aldehydes and derivatives thereof, are employed as the carbonyl compounds.

4. The process as claimed in one or more of claims 1 to 3, wherein the hydrogenation catalyst contains 20 to 90% by weight of nickel, based on the catalyst composition, and 1 to 30 parts by weight of aluminum oxide and 0.5 to 20 parts by weight of zirconium dioxide, in each case per 100 parts by weight of Ni, as a coprecipitate on a support material.

5. The process as claimed in one or more of claims 1 to 4, wherein the support material is active charcoal, alumina, pumice, γ-Al₂O₃, SiO₂, silica gel, kieselguhr and/or silicic earth, in particular SiO₂, silica gel, kieselguhr and/or silicic earth, preferably kieselguhr and/or SiO₂ in the form of precipitated silicic acid.

6. The process as claimed in one or more of claims 1 to 5, wherein the hydrogenation catalyst contains 6 to 80, in particular 15 to 65, preferably 35 to 50 parts by weight of support material per 100 parts by weight of Ni.

7. The process as claimed in one or more of claims 1 to 6, wherein the reaction is carried out in the liquid phase at 60 to 150, in particular 80 to 140, preferably 90 to 130, particularly preferably 100 to 125°C under a pressure of 0.1 to 25, in particular 1.0 to 15, preferably 2.0 to 10 MPa.

8. The process as claimed in claim 7, wherein the space velocity, expressed as the volume of liquid feed material/volume of catalyst x hour (V/Vh), is 0.6 to 2.0, in particular 0.8 to 1.6, preferably 1.0 to 1.5.

9. The process as claimed in one or more of claims 1 to 6, wherein the reaction is carried out in the gaseous phase at 60 to 150, in particular 80 to 140, preferably 90 to 130°C under a pressure of 0.05 to 2.0, in particular 0.1 to 1.2, preferably 0.15 to 1.0 MPa.

10. The process as claimed in claim 9, wherein the space velocity, expressed as the volume of liquid feed material/volume of catalyst x hour (V/Vh) is 0.2 to 1.5, in particular 0.3 to 1.2, preferably 0.5 to 1.0.

## Revendications

1. Procédé de préparation d'alcools par réaction de composés organiques carbonylés ou de mélanges contenant des composés organiques carbonylés avec l'hydrogène à température élevée et le cas échéant sous pression en présence d'un catalyseur d'hydrogénation, caractérisé en ce que l'on convertit les composés carbonylés ou leurs mélanges sur un catalyseur sur support contenant du nickel, de l'alumine et du dioxyde de zirconium à des températures de 60 à 150°C.

2. Procédé selon la revendication 1, caractérisé en ce que les dérivés carbonylés mis en oeuvre sont des cétones, des dérivés cétoniques, des aldéhydes et des dérivés aldéhydiques.

3. Procédé selon une ou plusieurs des revendications 1 à 2, caractérisé en ce que les dérivés carbonylés mis en oeuvre sont des aldéhydes et dérivés aldéhydiques, en particulier des aldéhydes aliphatiques et leurs dérivés.

4. Procédé selon une ou plusieurs des revendications 1 à 3, caractérisé en ce que le catalyseur d'hydrogénation contient de 20 à 90 % en poids de nickel, sur sa composition totale, avec 1 à 30 parties en poids d'alumine et 0,5 à 20 parties en poids de dioxyde de zirconium, dans les deux cas pour 100 parties en poids de nickel, à l'état de précipité commun sur une matière de support.

5. Procédé selon une ou plusieurs des revendications 1 à 4, caractérisé en ce que la matière de support consiste en charbon actif, alumine, ponce, γ-Al₂O₃, SiO₂, gel de silice, kieselguhr et/ou silice minérale, en particulier SiO₂, gel de silice, kieselguhr et/ou silice minérale, de préférence kieselguhr et/ou SiO₂ à l'état de silice précipitée.

6. Procédé selon une ou plusieurs des revendications 1 à 5, caractérisé en ce que le catalyseur d'hydrogénation contient de 6 à 80, plus spécialement de 15 à 65 et de préférence de 35 à 50 parties en poids de matière de support pour 100 parties en poids de nickel.

7. Procédé selon une ou plusieurs des revendications 1 à 6, caractérisé en ce que la réaction est effectuée en phase liquide à des températures de 60 à 150, plus spécialement de 80 à 140, et de préférence de 90 à 130 ou mieux encore de 100 à 125°C sous une pression de 0,1 à 25, plus spécialement de 1,0 à 15 et de préférence de 2,0 à 10 MPa.

8. Procédé selon la revendication 7, caractérisé en ce que la vitesse spatiale exprimée en volume de produit de départ liquide par volume de catalyseur et par heure (V/Vh) est de 0,6 à 2,0, plus spécialement de 0,8 à 1,6 et de préférence de 1,0 à 1,5.

9. Procédé selon une ou plusieurs des revendications 1 à 6, caractérisé en ce que la réaction est effectuée en phase gazeuse à des températures de 60 à 150, plus spécialement de 80 à 140, de préférence de 90 à 130°C et sous une pression de 0,05 à 2,0, plus spécialement de 0,1 à 1,2, et de préférence de 0,15 à 1,0 MPa.

10. Procédé selon la revendication 9, caractérisé en ce que la vitesse spatiale exprimée en volumes de produit de départ liquide par volume de catalyseur et par heure est de 0,2 à 1,5, plus spécialement de 0,3 à 1,2 et de préférence de 0,5 à 1,0.
